# EUROPEAN PATENT SPECIFICATION

(11) **EP 4 103 096 B1**
(45) Date of publication and mention of the grant of the patent: **29.01.2025**
(21) Application number: 21704019.5
(22) Date of filing: 09.02.2021
(51) Int. Cl.: A61B 34/37, A61B 46/10

(54) **STERILE BARRIER ASSEMBLY AND ROBOTIC SURGERY SYSTEM**
STERILE BARRIEREANORDNUNG UND ROBOTISCHES CHIRURGISCHES SYSTEM
ENSEMBLE BARRIÈRE STÉRILE ET SYSTÈME DE CHIRURGIE ROBOTISÉ

(30) Priority: 10.02.2020 IT 202000002545; 10.02.2020 IT 202000002536
(43) Date of publication of application: 21.12.2022
(60) Divisional application: 24221493.0
(73) Proprietor: Medical Microinstruments, Inc., Wilmington, DE 19801 (US)
(72) Inventor: SIMI, Massimiliano, 56121 Pisa (IT); LAZZARI, Giorgio, 56121 Pisa (IT)
(74) Representative: Crippa, Paolo Ernesto
(86) International application number: PCT/IB2021/051028
(87) International publication number: WO 2021/161161

(56) References cited:
- WO-A1-2011/143024
- WO-A1-2017/044884
- WO-A1-2019/150111
- US-A1- 2016 235 490
- US-A1- 2018 000 472
- US-A1- 2019 380 803

## Description

### . Field of the invention

. The present invention generally relates to surgical drapes for robotic surgery.

. It is an object of the present invention a sterile barrier assembly for robotic surgery.

. The present invention also relates to a robotic surgery system comprising at least one sterile barrier assembly.

.

### . Background

**.** Robotic surgery apparatuses are generally known in the art and typically comprise a robotic central tower (or robotic cart) and a plurality robotic arms extending from the central tower. Each robotic arm comprises a tele-operated robotic motorized positioning system (or manipulator) for moving a surgical end effector distally attached thereto, in order to perform surgical procedures to a patient. The patient typically lies on an operatory bed located in an operatory room, wherein sterilization is ensured in order to avoid bacterial contamination due to the non-sterile parts of the robotic apparatus.

**.** Usually, a surgical drape wraps parts of the robot in order to protect the sterile environment from contamination. The need of wrapping the robot arise from the fact that such robot must be used and re-used for several surgeries.

**.** For example, document US-2014-0158141 shows a pliable surgical drape designed to cover the robotic central tower and having a plurality of openings for each one of the robotic arms extending from the robotic cart so that the robotic arms are not covered by said surgical drape but are draped by means of a separate bag of surgical drape.

Therefore, the surgical drape flexible body may be provided with inserts, like rigid plastic plates, in order to form a connection interface with an active part of the robot. To this end, sterile adapters typically made of rigid plastic are integral with the drape to provide an attachment site for a surgical instrument and generally comprise movable parts in order to transmit actuation actions, such as a capstan driving action, to a surgical instrument detachably attached thereto for actuate an end effector of the surgical instrument. Examples of known sterile adapters can be found in documents WO-2009-061915 and WO-2019-006206**.**

**.** Drapes for covering microscope parts located within a operatory room are also known in the art. For example, document US-2019-0069969 discloses a tubular drape for fitting a pillar shaped terminal part of an electronic microscope, wherein a covering rigid glass is provided to the drape forming a cup-shaped end of the drape designed to cover the lens of the microscope. This solution of drape is therefore unsuitable to transmit an actuation action downstream the drape itself, and in this case downstream the lens of the microscope.

**.** Document WO-2017-064301 in the name of the same Applicant discloses a solution of robotic surgery apparatus wherein a single robotic arm extends from the robotic cart and comprises at the distal end thereof a pair of tele-operated robotic motorized manipulator systems, arranged in parallel and connected to the same horizontal link of the single robotic arm.

**.** Known surgical drapes can comprise also fixation strings that allow tying the surgical drape to close-fit an elongated portion of the robot, such as a robotic arm, thereby reducing the encumber of the surgical drape covering the robotic apparatus when in use. Moreover, it is also known to provide magnetic releasable coupling between parts of the robot apparatus and the margin of the surgical drape.

**.** Document WO-2018-189729 in the name of the same Applicant shows a sterile barrier solution involving stiffening elements attached thereto, with the purpose of transmitting linear actuation across the barrier itself. The surface of the stiffening elements may be rounded to guarantee a single point contact with the non-sterile actuators. Despite advantageous for several reasons, such a solution results difficult to assemble because of the requested alignment of the actuators of the tele-operated robotic motorized positioning system with the stiffening elements of the sterile barrier and, in turn, with the distal elements of the surgical instruments thereby actuated.

**.** Surgical drapes typically are in form of bags enclosing the robot, said bag having a closed end, and one or more pouches may further extend from the closed end of the bag to receive an extendable actuator of the robot, as shown for example in WO-2011-143024**.**

**.** It is also known to provide a rotative seal proximally to the surgical drape in order to drape both a robotic tower and a robotic arm that can pivot about an axis in respect of the robotic tower. An example of this type of solution is shown in document WO-2019-150111**,** wherein the rotatable seal is located at the proximal end of the pouch of the sterile bag draping robotic arm in order to pivotally connect the proximal end of said pouch to the drape portion draping the robotic tower. Other example of surgery systems having a rotatable seal are shown by documents US-2016-235490**,** WO-2017-044884**,** US-2019-380803 and US-2018-0000472**.** In these known solutions the rotatable seal is typically obtained by means of fitting/mounting a first surgical drape having a first ring defining a trough opening to a second surgical drape having an outer ring contouring the second surgical drape, after having connected the second surgical drape to a rotatable element of the robotic system.

**.** It is felt the need to provide a solution of sterile barrier with a rotatable seal of improved yet simplified assemblage.

**.** Therefore, the need is felt to provide a solution of surgical drape of simplified assemblage to a robotic surgery apparatus and at the same time of improved durability in respect of known solutions, without for these reasons inhibiting the transmission of an actuation action through and/or across the surgical drape.

**.** At the same time, it is felt the need to provide a solution of sterile barrier of improved durability in respect of known solutions without for this reason to limit the freedom of movement, during surgery, of a sterile surgical instrument distally connectable to the sterile barrier.

### . Solution

**.** It is a scope of the present invention to overcome the drawbacks mentioned with reference to the known art.

**.** These and other scopes are achieved by a sterile barrier assembly according to claim 1, as well as a a robotic surgery system according to claim 8

**.** Some preferred embodiments are the subject of dependent claims.

**.** According to an aspect of the invention, a sterile barrier assembly comprises a surgical drape having at least one drape opening and at least one connector contouring said at least one drape opening and suitable for coupling with a case encasing a non-sterile robotic manipulator.

**.** According to an aspect of the invention, the sterile barrier assembly further comprises at least one sterile adapter having a frame comprising a coupling device for coupling with at least one surgical instrument, wherein the at least one connector of the surgical drape and the at least one sterile adapter are made in separate pieces.

**.** According to an aspect of the invention, the at least one sterile adapter is suitable to pivot in respect of the at least one connector of the surgical drape and between the at least one connector of the surgical drape and the at least one sterile adapter it is provided a gap. The gap may be an annular gap and the gap prevents any direct contact of the frame of the sterile adapter with the connector of the sterile drape.

**.** The at least one connector of the surgical drape comprises a distal surface facing distally and said sterile adapter comprises a proximal surface facing proximally, and wherein said distal surface of the connector faces said proximal surface of the sterile adapter delimiting said gap. Thereby the proximal surface of the sterile adapter distally delimits the gap while the distal surface of the connector proximally delimits the same gap.

**.** The connector of the drape may be integral with the body of the surgical drape.

**.** The sterile adapter may comprise a skirt facing proximally said gap, said proximal surface may belong to said skirt.

**.** According to an aspect of the invention, a robotic surgery system comprising at least one of said sterile barrier assembly further comprises at least one non-sterile robotic manipulator comprising: a case and at least one roll motor, and at least one rolling body suitable to pivot in respect to said case. The roll motor may be operatively connected to the rolling body.

**.** According to an aspect of the invention, said at least one connector of the surgical drape of the sterile barrier assembly is coupled with said case of the non-sterile robotic manipulator, and said at least one sterile adapter of the sterile barrier assembly is coupled with said at least one rolling body of the non-sterile robotic manipulator either directly or indirectly, for example by interposition of a manipulator connecting element, so that said at least one roll motor is suitable to move at least said one rolling body together with said at least one sterile adapter of a pivoting movement with respect to said case of the non-sterile robotic manipulator.

**.** The at least one connector of the surgical drape of the sterile barrier assembly may be coupled detachably and rigidly to said case of the non-sterile robotic manipulator.

**.** The at least one sterile adapter of the sterile barrier assembly may be coupled detachably and rigidly to said at least one rolling body of the non-sterile robotic manipulator.

**.** The rolling body of the non-sterile robotic manipulator may comprise a manipulator connecting element and said sterile adapter may detachably engage with said manipulator connecting element, for example in an undercut manner such as by means of a bayonet coupling and/or a threaded coupling. The engagement between said sterile adapter and said manipulator connecting element may be located distally to said case and/or distally to said connector of the surgical drape. The manipulator connecting element may be located at least partially within said case while said sterile adapter may be located out of said case, and preferably distally to said case encasing said non-sterile robotic manipulator.

**.** According to an aspect of the invention, the sterile drape comprises a pair of drape openings and a pair of connectors and a pair of sterile adapters, wherein each connector contouring a respective drape opening, each connector being made in separate pieces with respect to a respective sterile adapter, each sterile adapter is suitable to pivot in respect of the respective connector of the surgical drape.

**.** According to an aspect of the invention, a robotic surgery system comprising a sterile barrier assembly of the above-described type having a pair of drape openings and a pair of connectors and a pair of sterile adapters further comprises a pair of non-sterile robotic manipulators, wherein each connector of the surgical drape is coupled to the case of a respective robotic manipulator and each sterile adapter of the sterile barrier assembly is coupled with the rolling body of a respective non-sterile robotic manipulator, so that each roll motor is suitable to move a respective rolling body together with said the respective sterile adapter of a pivoting movement with respect to the case of the respective non-sterile robotic manipulator.

**.** The body of the surgical drape may be made in single piece and may comprise a pair of pouches, each pouch delimiting a respective drape opening, each pouch being suitable for individually draping one non-sterile robotic manipulator of said pair, for example by means of attaching the connector of the respective pouch of the surgical drape to the case of the respective non-sterile robotic manipulator.

**.** According to the present disclosure, a method of mounting and/or dismounting a sterile barrier assembly of a robotic surgery system comprises the following steps of connecting and/or disconnecting at least one connector of a surgical drape to and/or from at least one case of a non-sterile robotic manipulator, and connecting and/or disconnecting a sterile adapter to a rolling body of the non-sterile robotic manipulator. The connection to the rolling body may be either direct or indirect, for example by means of interposition of a manipulator connecting element.

**.** Connecting the sterile adapter to the rolling body, either directly or indirectly for example by means of interposition of a manipulator connecting element, may comprise moving the sterile adapter proximally towards the rolling body, without touching the surgical drape and/or the connector of the surgical drape.

**.** Connecting the sterile adapter to the rolling body, either directly or indirectly for example by means of interposition of a manipulator connecting element, may comprise moving the sterile adapter proximally towards the rolling body, when the surgical drape is already attached by means of its connector to the case of a non-sterile robotic manipulator. Disconnecting the sterile adapter from the rolling body, either directly or indirectly for example by means of interposition of a manipulator connecting element, may comprise moving the sterile adapter distally from the rolling body, when the surgical drape is attached by means of its connector to the case of a non-sterile robotic manipulator. The method of mounting may therefore comprise firstly connecting at least one connector of a surgical drape to the at least one case of a non-sterile robotic manipulator, and subsequently connecting at least one sterile adapter to a rolling body of the non-sterile robotic manipulator having its case already connected to the connector of the surgical drape. The method of dismounting may therefore comprise firstly disconnecting at least one sterile adapter from the rolling body of the non-sterile robotic manipulator, and subsequently disconnecting the connector of the surgical drape from the at least one case of the at least one non-sterile robotic manipulator, which the sterile adapter is already disconnected from.

**.** Thanks to the proposed solutions, it is provided a surgical drape or surgical bag able to surround with a ring thereof a rolling body, which is rollable about an axis in respect of the surgical drape.

**.** Thanks to the proposed solutions, it is provided a sterile barrier assembly comprising a surgical drape and at least a sterile adapter pivotally associated to at least a distal opening of the surgical drape.

**.** Thanks to the proposed solutions, it is provided a robotic surgery system comprising a rolling body received within a case, wherein both the case and the rolling body are associated to the same sterile barrier assembly.

**.** Thanks to the proposed solutions, it is provided a method for mounting and/or a method for dismounting a sterile barrier assembly in a simple yet advantageous order.

### . Figures

**.** Further characteristics and advantages of the surgical drape, of the sterile barrier assembly, of the system and of the method will appear from the following description of preferred embodiments, which are given as examples and are not meant to be limiting, which makes reference to the attached figures, in which:

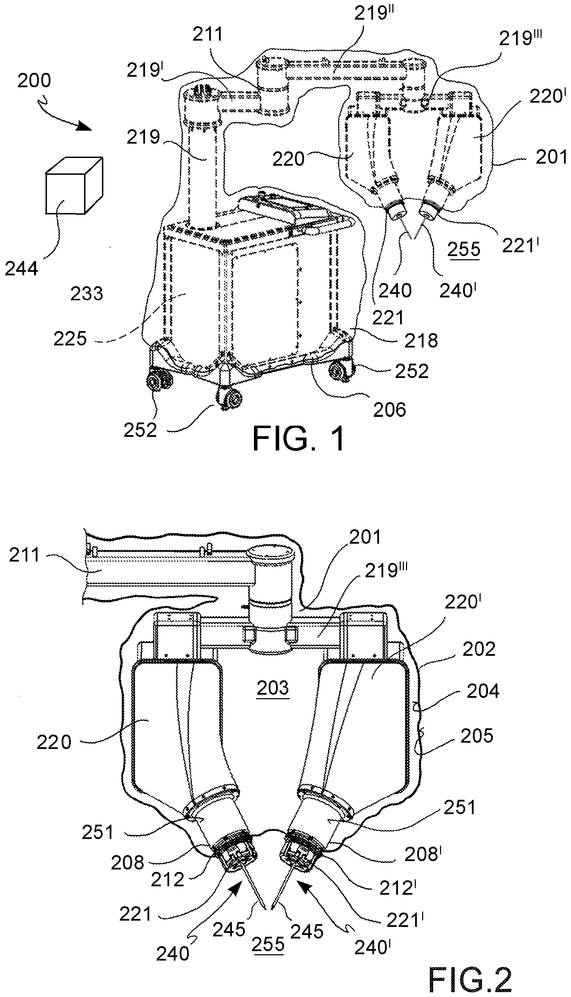

- figure 1 shows an axonometric view of a robotic surgery system comprising a sterile barrier assembly, according to an embodiment;
- figure 2 is a view of a robotic surgery system comprising a pair of manipulators and a sterile barrier assembly, according to an embodiment;

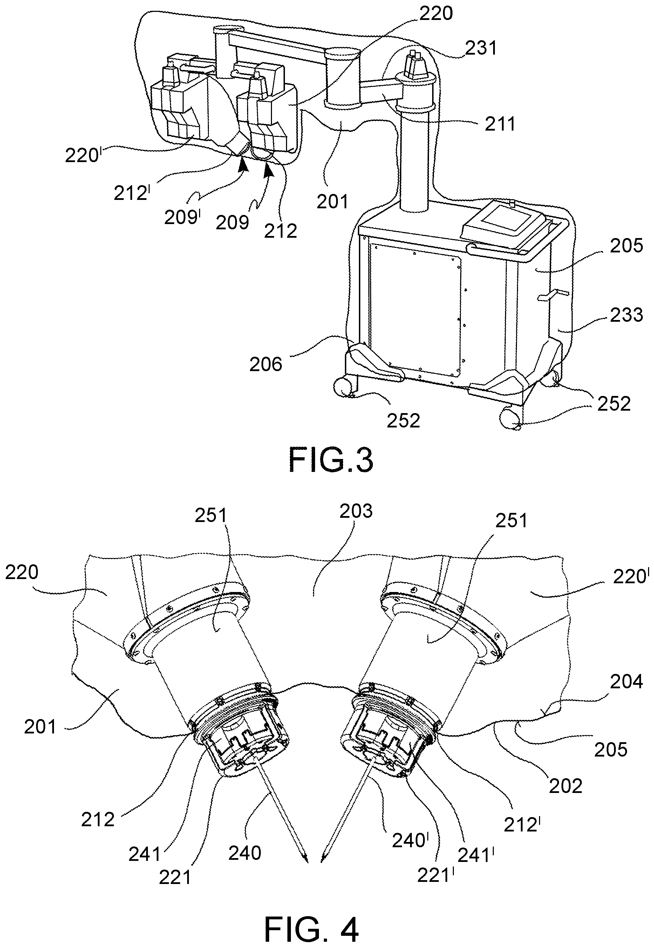

- figure 3 shows a robotic surgery system comprising a surgical drape, according to an embodiment;
- figure 4 an enlarged view of a detail of the system and the assembly shown in figure 2;
- figure 5 shows diagrammatically in axonometric view a surgical drape, according to an embodiment;
- figure 6 shows in axonometric view a sterile adapter of a sterile barrier assembly, according to an embodiment;

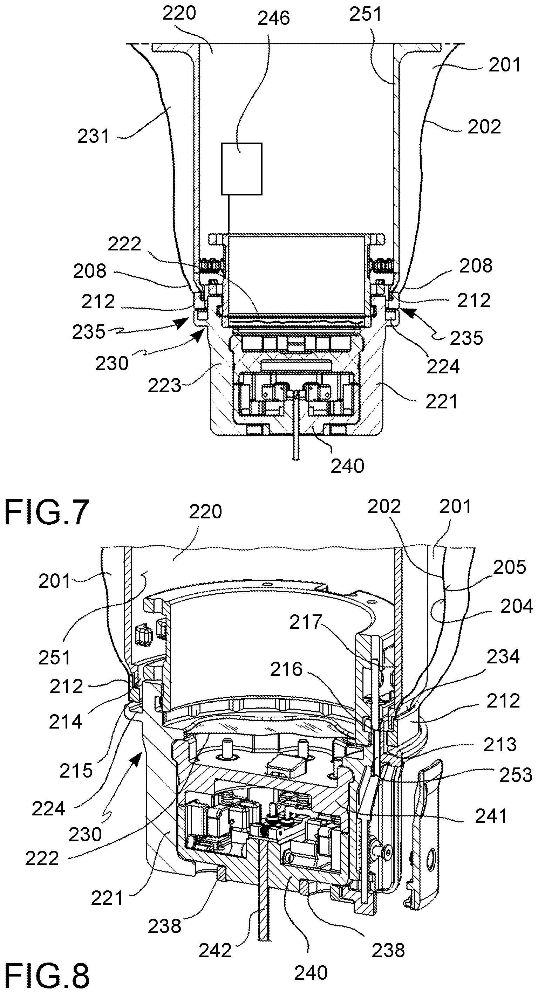

- figure 7 is a cross-section showing a sterile barrier assembly, according to an embodiment, and showing diagrammatically a motor of a robotic surgery system;
- figure 8 is a cross section in axonometric view showing the sterile barrier assembly of figure 7 coupled to a robotic surgery system;

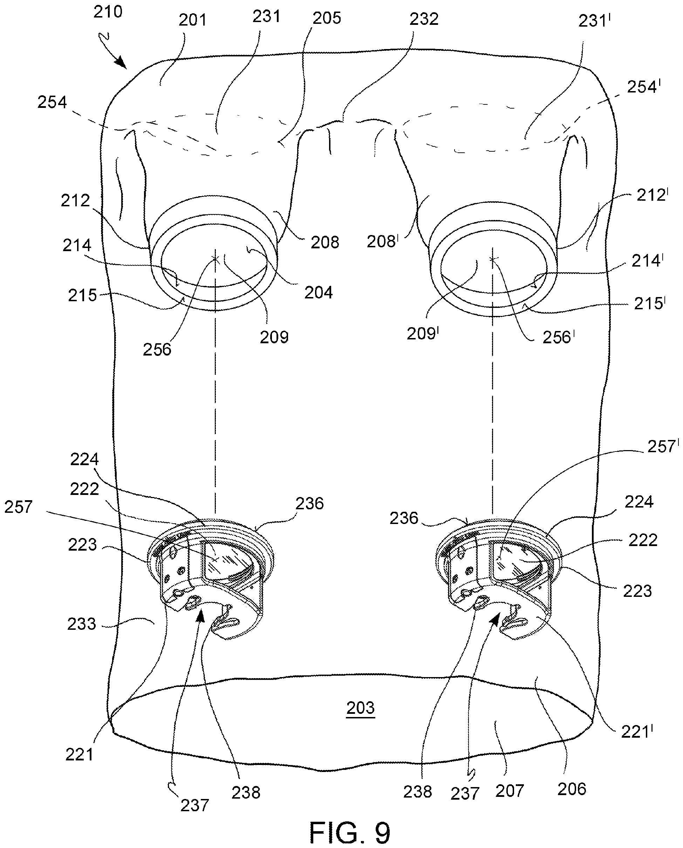

- figure 9 shows a sterile barrier assembly, according to an embodiment;
- figure 10 shows diagrammatically a cross-section of the system and the assembly shown in figure 4;
- figure 11 is an axonometric view of a manipulator of a robotic surgery system coupled to a surgical drape, according to an embodiment, wherein some parts of the system are drawn transparent for sought of clarity.
- figure 12 is a view of a robotic surgery system comprising a pair of manipulators and a pair of respective sterile barrier assemblies, according to an embodiment;
- figure 13 shows an axonometric view with separate parts of a portion of a robotic surgery system, according to an embodiment;
- figure 14 shows a cross-section of a portion of a robotic surgery system, according to an embodiment;

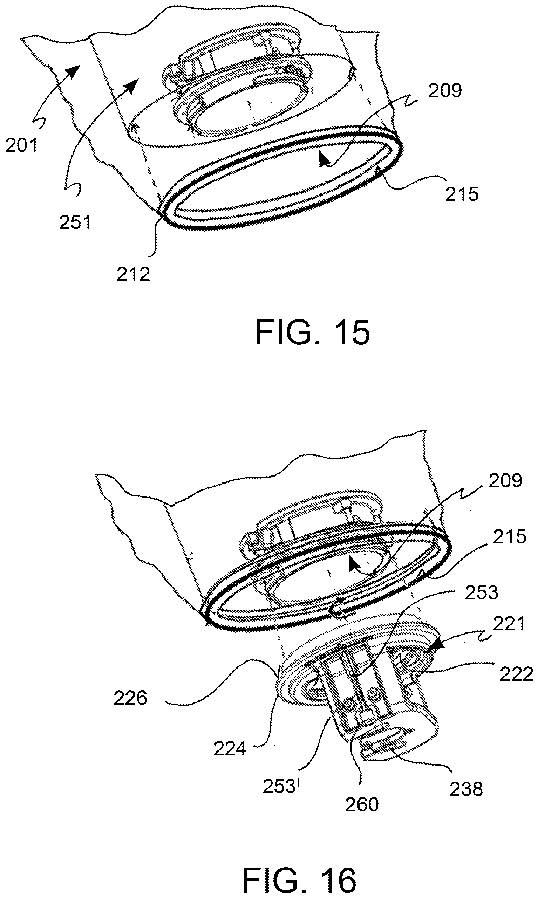

- figures 15 and 16 show some steps of a method according to a mode of operation.

### . Detailed description of some embodiments

**.** According to a general embodiment, it is provided a sterile barrier assembly 210 for a robotic surgery system 200.

**.** The sterile barrier assembly 210 comprises a surgical drape 201 or sterile drape 201 having at least one drape opening 209, 209'.

**.** The surgical drape 201 may be configured as a bag designed to fit a main portion of a robotic surgery system 200.

**.** A robotic surgery system 200 may comprise a robotic cart 225 and at least one robotic arm 211 extending from the cart 225 and defining a proximal-distal direction z-z. Said at least one robotic arm 211 may comprise a plurality of arm links 219, 219', 219", 219‴ connected one another forming a kinematic chain. One of said arm links 219, 219', 219", 219‴ may be connected to at least two non-sterile motorized robotic manipulators 220, 220', suitable to manipulate a robotic end effector, for example a wrist, within a patient's body along a plurality of degrees of freedom. According to an embodiment, at least one roll motor 246, is provided suitable to move said rolling body 230, 230' of a rolling movement about a rolling axis Z-Z in respect of a case 251, 251' of the non-sterile robotic manipulator 220, 220'.

**.** Said surgical drape 201 may comprise a body 202, and said body 202 delimits a drape cavity 203. According to a preferred embodiment, said body 202 is made of a flexible sheet of plastic material, for example polyethylene. Preferably, the term "flexible" used herein means that the body of the drape is loose, for example it is a flexible sheet. According to an embodiment, the body of the sterile drape 201 is made of paper, woven fabric, non-woven fabric, and/or any suitable material for surgical drape 201.

**.** According to a preferred embodiment, said body 202 of the surgical drape 201 is in single piece. Preferably, said body 202 is a single piece of flexible sheet.

**.** According to a preferred embodiment, said surgical drape 201 may comprise a first inner surface 204 facing the drape cavity 203, and a second outer surface 205 facing away from the cavity 203. Thereby, said first inner surface 204 and said second outer surface 205 are opposite one another in respect of the flexible body 202 of the surgical drape 201.

**.** Preferably, said surgical drape 201 comprises a proximal opening edge 206 delimiting a proximal opening 207 of the surgical drape 201 for accessing said drape cavity 203, wherein said proximal opening 207 is preferably a through opening.

**.** According to a preferred embodiment, said proximal opening edge 206 is associated to a proximal coupling interface 218 suitable for forming a coupling with a portion of a robotic surgery system 200. According to an embodiment, said proximal coupling interface 218 of the proximal opening edge 206 of said surgical drape 201 forms a magnetic coupling with a part of the cart 225 of a robotic surgery system 200. For example, said proximal coupling interface 218 comprises a permanent magnet. For example, said proximal coupling interface 218 comprises a ferromagnetic portion suitable to form a magnetic coupling with a magnetic part of the cart 225. According to an embodiment, said proximal coupling interface 218 of the proximal opening edge 206 of said surgical drape 201 comprises an elastic strip forming an elastic pressure action on the cart 225 of the robotic surgery system 200. According to an embodiment, said proximal coupling interface 218 of the proximal opening edge 206 of said surgical drape 201 comprises straps for tying to said cart 225.

**.** Preferably, said surgical drape 201 comprises at least one distal opening edge 208, 208' delimiting a respective distal opening 209, 209' for accessing said drape cavity 203.

**.** Said at least one distal opening 209, 209' is preferably a distal through opening 209, 209'.

**.** According to an embodiment, said drape cavity 203 is a through cavity extending between and leading through said proximal opening 207 and said at least one distal opening 209, 209'. Thereby, the robotic surgery system 200 may be dressed at least partially with said surgical drape 201 by firstly fitting said proximal opening edge 206 of the surgical drape 201 around a distal end of a robotic arm 211 of the robotic surgical system 200, then moving proximally said proximal opening edge 206 of the surgical drape 201 thereby unfolding the surgical drape 201 and fitting said robotic arm 211 by the body 202 of the surgical drape 201, then coupling said at least one distal opening edge 209, 209' to a respective part of the robotic arm 211 of the robotic surgery system 200.

**.** Said sterile barrier assembly 210 further comprises at least one connector 212, 212' contouring said at least one drape opening 209, 209' and suitable for coupling with said case 251, 251' of said non-sterile robotic manipulator 220, 220'.

**.** Preferably, the surgical drape 201 comprises at least one annular connector 212, 212' associated to a respective distal opening edge 208, 208' of the flexible body 202. According to an embodiment, said at least one annular connector 212, 212' is a ring connector 212, 212'.

**.** Thereby, when said first distal opening edge 208 and said second distal opening edge 208' are provided, a respective first annular connector 212 is associated to said first distal opening edge 208 to contour said first distal opening 209, and a respective second annular connector 212' is associated to said second distal opening edge 208' to contour said second distal opening 209'.

**.** Said at least one annular connector 212, 212' comprises an annular rim 213, 213' facing a respective distal annular opening 209 or 209' and having an annular shape, so that said at least one distal opening 209, 209' is contoured by said annular rim 213, 213'. Thereby, the at least one distal opening 209, 209' of the surgical drape 201 has an annular contour. Thus, the at least one distal opening 209, 209' may have the shape of a circle.

**.** According to a preferred embodiment, said at least one annular connector 212, 212' is more rigid than the body 202 of the surgical drape 201. According to a preferred embodiment, said at least one annular connector 212, 212' is rigid so that a manual action applied thereto is unsuitable to elastically deform the annular connector 212, 212' without creep it and/or break it. According to a preferred embodiment, said annular rim 213, 213' of the at least one annular connector 212, 212' is rigid so that to define an annular contour of the at least one distal opening 209, 209'.

**.** Said sterile barrier assembly 210 comprises at least one sterile adapter 221, 221' having a frame 223 comprising a coupling device 237 for coupling with at least one sterile surgical instrument 240, 240'.

**.** Said at least one connector 212, 212' of the surgical drape 201 and the at least one sterile adapter 221, 221' are made in separate pieces.

**.** Said at least one sterile adapter 221, 221' is suitable to pivot or to roll in respect of the at least one connector 212, 212' of the surgical drape 201.

**.** Advantageously, between the at least one connector 212, 212' of the surgical drape 201 and the at least one sterile adapter 221, 221' it is provided a gap 235, 235'.

**.** Thereby, said gap 235, 235' forms together with said at least one connector 212, 212' and with said at least one sterile adapter 221, 221' a rotatable seal, which avoids to generate rotative friction onto the at least one connector 212, 212' and the respectively pivotally associated sterile adapter 221, 221', while preserving sterility of a sterile operatory field 255, in other words preventing the sterile operatory field 255 from being contaminated by the non-sterile parts of the robotic surgical system 200, which is draped. Thanks to the provision of said gap 235, 235' the sterile adapter 221, 221' is not connected directly to the connector 212, 212' of the surgical drape 201.

**.** According to a preferred embodiment, said body 202 of the surgical drape 201 further comprises a first distal opening edge 208 and a second distal opening edge 208', each of said first distal opening edge 208 and of said second distal opening edge 208' delimits a respective distal through opening 209, 209'. Thereby, a first distal through opening 209 is delimited by said first distal opening edge 208 and a second distal through opening 209' is delimited by said second distal opening edge 208'. Thereby, when said first annular connector 212 and said second annular connector 212' are provided, each of said first and second annular connectors 212 and 212' comprises an annular rim 213, 213', so that a first annular rim 213 is associated to said first annular connector 212 and a second annular rim 213' is associated to a second annular connector 212'. Preferably, each of said annular rim 213, 213' of the at least one annular connector 212, 212' defines a connection ring suitable for connecting to a robotic surgery system 200.

**.** The provision of a pair of distal openings 209 and 209' each associated to a respective annular connector 212 and 212' allows to use a single surgical drape 201 to drape a pair of robotic arms and a portion of the cart 225 of the robotic surgery system 200.

**.** The provision of such at least one annular connector 212, 212' allows to receive within said annular rim 213, 213' thereof a rolling body 230, 230' that can roll about an axis Z-Z in respect of the body 202 of the surgical drape 201 and/or in respect of said at least one annular connector 212, 212'.

**.** According to an embodiment, with the terminology "a rolling body 230, 230' that can roll about an axis" also encompasses the case wherein the rolling movement is a relative movement between said rolling body 230, 230' of the robotic surgery system 200 and said annular connector 212, 212' of the surgical drape 201.

**.** Thereby, said relative rolling movement may require the annular connector 212, 212' to roll and/or pivot about an axis and the rolling body 230, 230' to be substantially stationary, defining a relative rolling movement.

**.** According to an embodiment, with the terminology "a rolling body 230, 230' that can roll about an axis" also encompasses the case wherein the rolling body 230, 230' can pivot about an axis in respect of the body 202 of the surgical drape 201 and/or in respect of said at least one annular connector 212, 212'.

**.** According to an embodiment, with the terminology "a rolling body 230, 230' that can roll about an axis" also encompasses the case wherein the rolling body 230, 230' can roll about a movable axis in respect of the body 202 of the surgical drape 201 and/or in respect of said at least one annular connector 212, 212', for example a spherical joint and the like.

**.** The provision of a pair of annular connectors 212 and 212' allows to receive a pair of rolling bodies 230, 230', each one of said pair of rolling bodies 230, 230' being received within a respective annular rim 213, 213' in such way that each one of said pair of rolling bodies 230 and 230' can roll about an axis z-z in respect of the body 202 of the surgical drape 201 and/or in respect of a respective annular rim 213, 213' of annular connector 212, 212'. According to an embodiment, each one of said pair of rolling bodies 230 and 230' can roll about an axis z-z independently from the other rolling body of said pair of rolling bodies 230, 230'. The respective roll axis z-z of each one of said pair of rolling bodies 230, 230' may be parallel one another, although according to a preferred embodiment are not parallel, and more preferably they converge one towards the other,

**.** Said at least one rolling body 230. 230' received within said at least one annular connector 212, 212' in said at least one distal opening 209, 209' of the surgical drape 201 may have a substantially cylindrical geometry extending around a roll axis that is either substantially stationary or in turn rotatable around a further axis in respect of said at least one annular connector 212, 212'. Alternatively said at least one rolling body 230. 230' received within said at least one annular connector 212, 212' in said at least one distal opening 209, 209' of the surgical drape 201 may have a substantially spherical geometry suitable for rolling around a roll axis that is either substantially stationary or movable in respect of said at least one annular connector 212, 212'.

**.** According to a preferred embodiment, said at least one annular connector 212, 212' is integral with said flexible body 202 of the surgical drape 201. Thereby, said at least one annular connector 212, 212' cannot roll in respect of said body 202 of the surgical drape 201. Not necessary said body 202 of the surgical drape 201 is made as single piece with said at least one annular connector 212, 212', although according to an embodiment they are made in single piece of material. According to an embodiment, said at least one annular connector 212, 212' is glued and/or fixed and/or clamped and/or pinched and/or thermoformed and/or otherwise secured to said at least one distal opening edge 208, 208' of said body 202 of the surgical drape 201. Preferably. the connector 212, 212' is in turn detachably connected to the case 251, 251' encasing said robotic manipulator 220, 220'.

**.** According to an embodiment, the sterile barrier assembly comprises at least one pouch 231, 231' having a lateral wall formed by said body 202 of the surgical drape 201 and extending proximally in respect to said at least one distal opening edge 208, 208' and preferably comprising said at least one distal opening edge 208, 208'. Thereby said at least one pouch 231, 231' is suitable for draping at least partially said robotic arm 211 and preferably said at least one manipulator 220, 220' of the robotic surgery system 200.

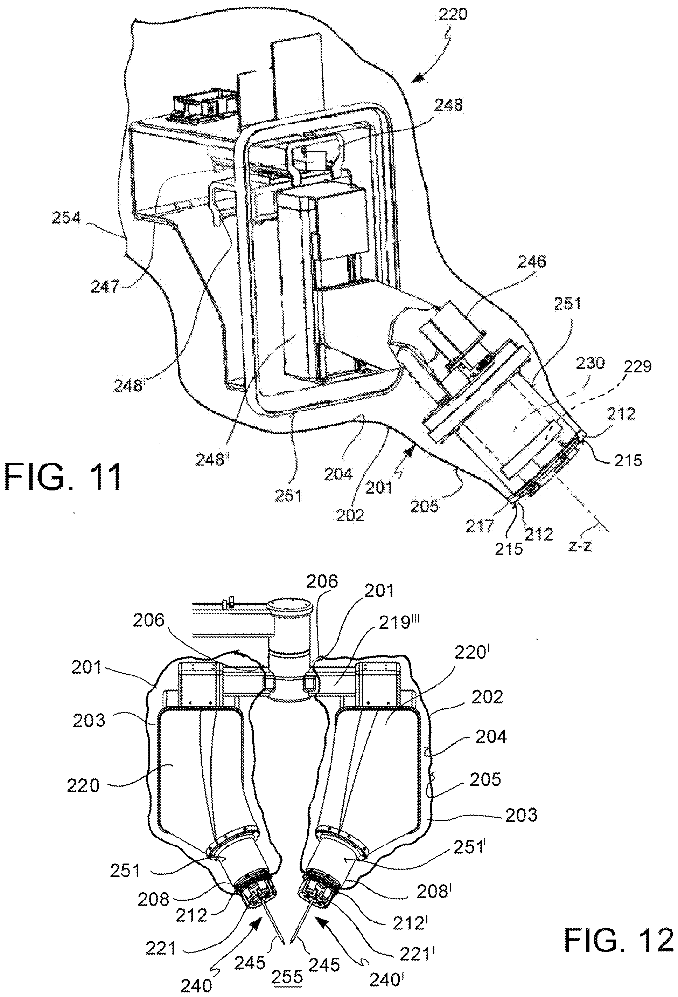

**.** According to an embodiment, said proximal opening edge 206 of the surgical drape 201 is suitable for forming a coupling with a portion of the non-sterile robotic manipulator 220 or 220' of the robotic surgery system 200. Thereby, the robotic surgery system 200 may comprise more than one surgical drapes 201 and/or more than one sterile barrier assemblies 210, as shown for example in **figure 12****.** Thereby, the robotic arm 211 comprising two non-sterile robotic manipulators 220, 220' is draped by two surgical drapes 201 each forming a sterile barrier assemblies 210.

**.** Preferably, said at least one pouch 231, 231' is formed by a sleeve of said surgical drape 201 and one of said sterile adapter 221, 221' and said rotatable seal formed by said gap 235, 235'. Preferably, each pouch 231, 231' has at least one respective drape opening 209, 209', and an opposite proximal pouch opening 254, 254', wherein the rotatable seal is distal to said proximal pouch opening 254, 254'. Distally to the rotatable seal preferably there is the sterile adapter 221, 221'.

**.** According to an embodiment, said surgical drape 201 comprises a pair of said pouches 231, 231', preferably each comprising a respective distal opening edge 208, 208'. According to an embodiment, said body 202 of said surgical drape 201 comprises a single pouch 231 for covering a robotic arm 211, wherein said single pouch 231 comprises a pair of distal opening edges 208 and 209'. According to an embodiment, said body 202 of the surgical drape 201 comprises a bridge portion 232 connecting said distal opening edges 208 and 208' one another. Preferably, said bridge portion 232 of the body 202 of the surgical drape 201 connects said pair of tubular portions 231, 231' one another forming a shared root portion.

**.** According to an embodiment, said at least one annular connector 212, 212' forms the free end of the body 202 of the surgical drape 201. Preferably, said at least one annular connector 212, 212' forms the free end of a respective pouch 231, 231' thereof.

**.** According to an embodiment, said body 202 of the surgical drape 201 comprises a base portion 233 comprising said at least one proximal opening edge 206. Thereby, said base portion 233 designed to drape a robotic cart 225. According to an embodiment, said at least one pouch 231, 231' extends from said base portion 233 of the body 202 of the surgical drape 201. The provision of said base portion 233 and of said at least one pouch 231, 231', allows said body 202 of a single surgical drape 201 to drape both a robotic cart 225 and at least one a robotic arm 211 of a robotic surgery system 200.

**.** The provision of a surgical drape 201 having body 202 comprising at least a pouch 231, 231' to drape at least robotic arm 211 and a base portion 233 to drape a robotic cart 225, wherein said at least one pouch 231, 231' is associated to a pair of annular connectors 212, 212' allows to drape with a single surgical drape 201 a robotic surgery system 200 comprising at least two robotic manipulators 220, 220' attached distally to at least one robotic arm 211, prefefrably wherein both manipulators 220, 220' are attached to the same link of the robotic arm 211, wherein preferably the robotic arm is in turn attached to a robotic cart 225.

**.** According to an embodiment, said annular rim 213, 213' of the at least one annular connector 212, 212' comprises an inner annular surface 214, 214' facing a respective distal opening 209, 209'. Thereby, when a pair of annular connectors 212 and 212' is provided, a pair of respective inner annular surfaces 214, 214' is provided. According to an embodiment, each of said inner annular surfaces 214, 214' faces a respective roll axis. According to an embodiment, each of said inner annular surfaces 214, 214' defines a ring that contours a respective distal opening 209, 209'. According to an embodiment, each of said inner annular surfaces 214, 214' is substantially parallel to the proximal distal direction Z-Z. According to an embodiment, each of said inner annular surfaces 214, 214' faces a respective rolling body 230, 230'.

**.** According to an embodiment, said at least one annular connector 212, 212' of the surgical drape 201, and preferably each of said annular connectors 212, 212', comprises a distal annular surface 215, 215' facing distally. Thereby, the distal annular surface 215, 215' of the annular connector 212, 212' faces away from the cavity 203 of the surgical drape 201 along the proximal-distal direction Z-Z. According to an embodiment, said at least one annular connector 212, 212' of the surgical drape 201, and preferably each of said annular connectors 212, 212', comprises a proximal annular surface 234 facing proximally. Thereby, said proximal annular surface 234 and said distal annular surface 215, 215' face opposite one another in respect of said annular connector 212, 212'. Thereby, said proximal annular surface 234 faces towards the body 202 of the surgical drape 201. According to an embodiment, said proximal annular surface 234 faces towards said cavity 203 of the surgical drape 201 and faces towards said first inner surface 204 of the body 202 of the surgical drape 201. Said proximal annular surface 234 may face away from the cavity 203 of the surgical drape 201 if faces towards the second outer surface 205 of the body 202 of the surgical drape 201.

**.** According to a preferred embodiment, the distal annular surface 215, 215' of the annular connector 212, 212' of the surgical drape 201 faces distally the proximal surface 226 of the frame 223 and/or the skirt 224 of the sterile adapter 221, 221', so that the distal annular surface 215, 215' of the annular connector 212, 212' of the surgical drape 201 and the proximal surface 226 of the frame 223 and/or the skirt 224 of the sterile adapter 221, 221' delimit said annular gap 235, 235'. Thereby the annular connector 212, 212' of the surgical drape 201 and the sterile adapter 221, 221' are not directly connected to each other. Bearings 246 may be provided preferably interposed between the manipulator connecting element 229, 229' and the case 251, 251' of the robotic manipulator 220, 220', therefore it is avoided the provision of bearings 246 in contact with both the sterile adapter 221, 221' and the connector 212, 212' of the drape 201.

**.** According to an embodiment, said at least one annular connector 212, 212' comprises a coupling device 216 suitable to couple the surgical drape 201 to a counter-coupling portion 217 of a robotic surgery system 200.

**.** According to an embodiment, said counter-coupling portion 217 is a portion of a robotic arm 211 of the surgical system 200 that is preferably located near a distal end of said robotic arm 211. According to an embodiment, said counter-coupling portion 217 is a portion of a manipulator 220, 220' of the robotic surgery system 200.

**.** According to an embodiment, said annular coupling device 216 of the surgical drape 201 comprises an elastic element, for example a clip, suitable for snap-fit engaging said counter-coupling portion 217 of the robotic arm 211 of the robotic surgery system 200. According to an embodiment, said coupling device 216 of the surgical drape 201 comprises a magnetic coupling element suitable for forming a magnetic coupling with said counter-coupling portion 217 of the robotic arm 211 of the robotic surgery system 200. According to an embodiment, said coupling device 216 of the surgical drape 201 comprises a threaded interface suitable for being screwed to said counter-coupling portion 217 of the robotic arm 211 of the robotic surgery system 200. According to an embodiment, said coupling device 216 of the surgical drape 201 comprises a bayonet coupling interface suitable for forming a bayonet-type engagement with said counter-coupling portion 217 of the robotic arm 211 of the robotic surgery system 200.

**.** According to a general embodiment, it is provided a sterile barrier assembly 210 comprising at least one surgical drape 201 according to any one of the embodiments previously described.

**.** According to an embodiment, said at least one sterile adapter 221, 221' comprises at least a second sheet 222, or membrane 222. Said second sheet 222 is preferably a single continuous flexible sheet of plastic material. Said second sheet 222 is preferably made of a drape material suitable for draping a robotic surgery system 200. For example, said second sheet 222 is made of the same material of the body 202 of the surgical drape 201. According to a preferred embodiment, said at least one sterile adapter 221, 221' is coaxial with said at least one annular rim 213, 213' of said at least one annular connector 212, 212' of the surgical drape 201. According to a preferred embodiment, said at least one sterile adapter 221, 221' comprises at least one substantially rounded rim 223.

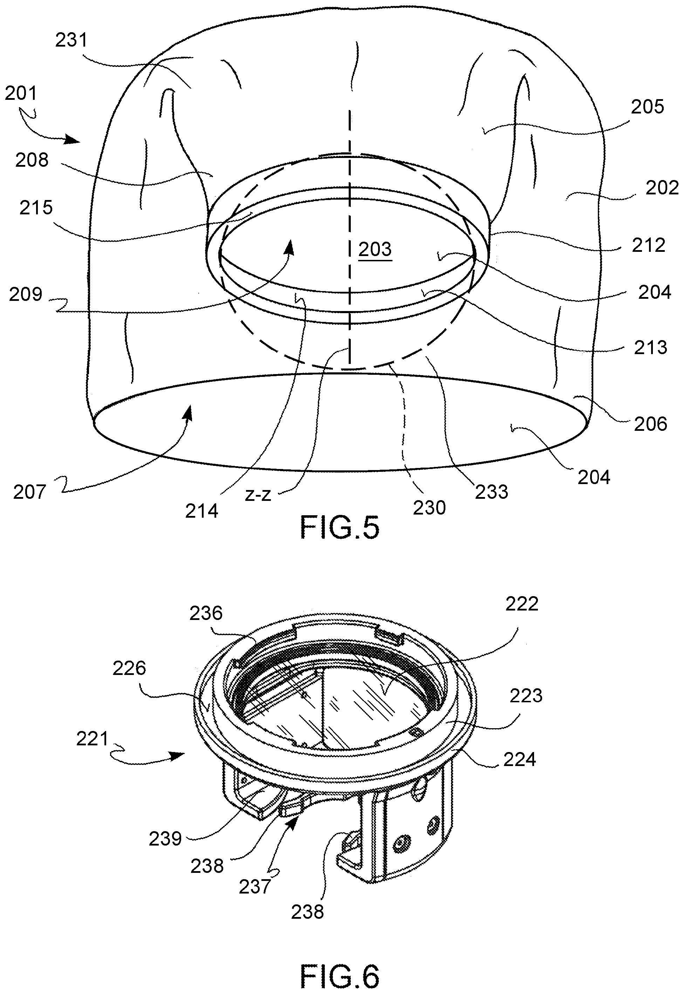

**.** According to a preferred embodiment as shown for example in **figure 6****,** said second sheet 222 has a substantially disc shape. According to a preferred embodiment, said rounded rim 223, or frame 223, of the at least one sterile adapter 221, 221' acts as constraining frame for the second sheet 222. According to a preferred embodiment, said at least one sterile adapter 221, 221' has a substantially cylindrical shape. According to a preferred embodiment, said at least one sterile adapter 221, 221' has a substantially spherical shape.

**.** According to a preferred embodiment, said at least one connector 212, 212' of the surgical drape 201 has an annular rim 213, 213' and said at least sterile adapter 221, 221' has an annular frame so that said gap 235, 235' is an annular gap.

**.** According to a preferred embodiment, the annular rim 213, 213' and the annular frame 223 of the sterile adapter 221, 221' are arranged coaxially. Preferably, said substantially rounded rim 223 or frame 223 of said sterile adapter 221, 221' is coaxial with the annular rim 213, 213' of said at least one annular connector 212, 212'. Thereby, the sterile adapter 221, 221' may roll about an axis in respect of a respective annular connector 212, 212' having the annular rim 213, 213' coaxial with said rounded rim 223 of the sterile adapter 221, 221'.

**.** According to a preferred embodiment, the at least one sterile adapter 221, 221' is suitable to pivot in respect of the at least one connector 212, 212' of the surgical drape 201 about an axis z-z, which passes through the geometric centre 257, 257' of the connector 212, 212'.

**.** According to an embodiment, said at least one sterile adapter 221, 221' is integrally coupled to a rolling body 230, 230' of the robotic surgery system 200. Thereby, said at least one sterile adapter 221, 221' of the sterile barrier assembly 210 can roll about an axis z-z in respect of the surgical drape 201 and/or in respect of said at least one annular connector 212, 212'.

**.** According to an embodiment, said at least one sterile adapter 221, 221' is coupled to said annular connector 212, 212' of the surgical drape 201 in such way that a relative rolling movement is allowed. Thereby, the sterile adapter 221, 221' acts as rolling body 230, 230'.

**.** According to an embodiment, said second sheet 222 of the at least one sterile adapter 221, 221' has form of a bag defining a proximal opening of second sheet 228 facing said at least one distal opening 209, 209' of the surgical drape 201, and preferably coaxial with said annular rim 213, 213' of said at least one annular connector 212, 212' of the surgical drape 201. According to an embodiment, said rounded rim 223 of the at least one sterile adapter 221, 221' comprises an engaging interface 226 that engages a counter-engaging interface 227 of the at least one annular connector 212, 212' of the surgical drape 201. Bearings may be provided between said engaging interface 226 and said counter-engaging interface 227.

**.** According to a preferred embodiment, said at least one sterile adapter 221, 221' comprises at least one proximal coupling device 236 suitable to form a detachable integral connection with a rolling body 230, 230' of the robotic surgery system 200. According to an embodiment, said proximal coupling device 236 is coupled with a manipulator connecting element 229, 229' comprising proximal connecting means for connecting the manipulator connecting element 229, 229' and the sterile adapter 221, 221' to a robotic non-sterile manipulator 220, 220'. Preferably, said manipulator connecting element 229, 229' acts as or is a rolling body 230, 230'.

**.** According to a preferred embodiment, said manipulator connecting element 229, 229' is a component of the robotic surgery system connecting said sterile adapter 221, 221' to the non sterile robotic manipulator. Thereby, the sterile adapter 221, 221' is connected in a detachable manner to the manipulator connecting element 229, 229'. In this way, the sterile adapter 221, 221' avoids to connect to the surgical drape, in other words the sterile adapter 221, 221' avoids to connect to the annular rim 213, 213' of the surgical drape as well as to the connector 212, 212' of the surgical drape. Thereby, the connector 212, 212' of the surgical drape does not extert any structural holding action on the sterile adapter 221, 221', and rather the connector 212, 212' of the surgical drape engages with the case 251, 251' while the sterile adapter 221, 221' engages with the manipulator connecting element 229, 229' which is in turn engaged to the robotic manipulator, so that the sterile adapter 221, 221' and the manipulator connecting element 229, 229' together rotate with respect to the connector 212, 212' of the surgical drape and with the case 251, 251' as well.

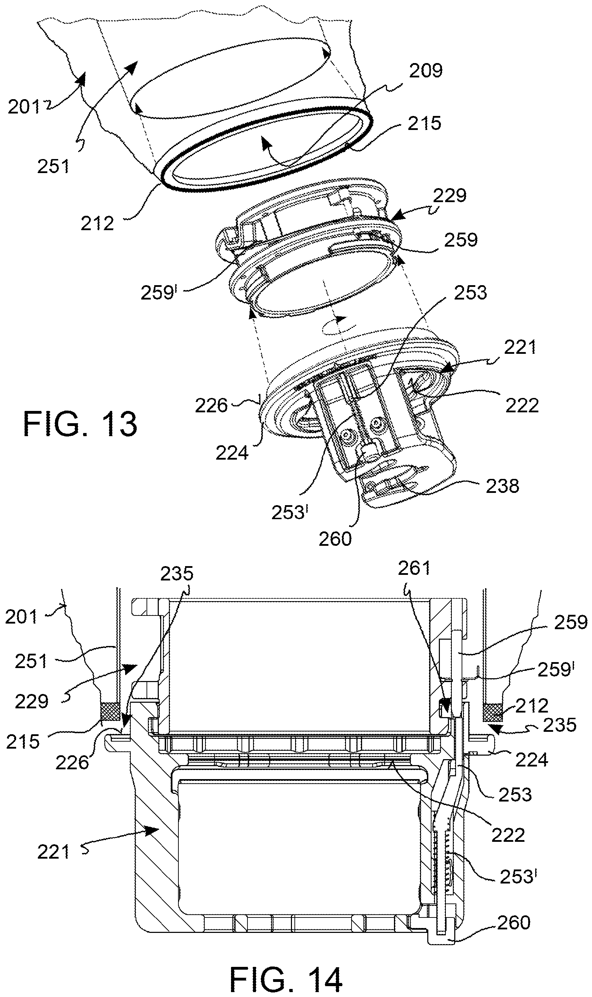

**.** According to an embodiment, the sterile adapter 221, 221' comprises a spring-loaded engaging pin 253 and the manipulator connecting element 229 ,229' comprises a respective spring-loaded counter-engaging pin 259, to flag when the sterile adapter 221, 221' is coupled to the manipulator connecting element 229, 229'. When the sterile adapter 221, 221' is not coupled to the manipulator connecting element 229, 229', the spring-loaded counter-engaging pin 259 of the manipulator connecting element 229, 229' abuts against a proximal surface of the sterile adapter 221, 221', such as a part of the proximal coupling device 236 of the sterile adapter. When the sterile adapter 221, 221' is coupled to the manipulator connecting element 229, 229', the spring-loaded engaging pin 253 counter-engaging pin 259 are aligned and abut one toward the other, the counter engaging pin 259 of the manipulator connecting element 229, 229' penetrates in a respective seat 261 and acts as anti-rotation element that avoids relative rotation of the adapter 221 ,221' and the manipulator connecting element 229, 229', as shown for example in figure 14: in other words, the counter engaging pin 259 of the manipulator connecting element 229, 229' when in said seat 261 of the sterile adapter 221, 221' abuts circumferentially against the walls of said seat 261 and avoids relative rotation between adapter 221, 221' and connecting element 229, 229'. In this way, the counter engaging pin 259 of the manipulator connecting element 229, 229' pushes distally said spring-loaded engaging pin 253, thereby determining a flagging protrusion 260 to protrude distally from the distal portion of the sterile adapter 221, 221'. In the example of figure 13 the spring-loaded engaging pin 253 is associated to coil spring 253' and the spring-loaded counter-engaging pin 259 is associated to flat spring 259'. Preferably the elastic action of the flat spring 259' overcomes the elastic action of the coil spring 253'.

**.** According to an embodiment, in order to disengage the sterile adapter 221, 221' from the manipulator connecting element 229, 229', the flagging protrusion 260 shall be pressed, thereby disengaging the spring-loaded counter-engaging pin 259, so that the adapter 221, 221' is able to rotate to disengage from the manipulator connecting element 229, 229'.

**.** According to a preferred embodiment, said sterile barrier assembly 210 further comprises at least one labyrinth seal device comprising said gap 235, 235' forming a rotatable seal. Preferably, said labyrinth seal device provides a tortuous path that prevents mutual contamination across the length of the tortuous path. An aspiration device may be functionally associated to the labyrinth seal to create a depression that cooperates with said labyrinth seal device to prevent contamination towards the distal side of the sterile barrier assembly 210. According to a preferred embodiment, said at least one labyrinth seal device extends around the roll axis z-z. According to a preferred embodiment, said at least one labyrinth seal device contours said at least one drape opening 209, 209' and contours the frame 223 of said sterile adapter 221, 221'.

**.** According to a preferred embodiment wherein said sterile barrier assembly 210 comprises a pair of sterile adapters 221 and 221', a first gap 235 is provided between said first distal opening edge 208 of the surgical drape 201 and a first sterile adapter 221, and a second gap 235' is provided between said second distal opening edge 208' of the surgical drape 201 and a second sterile adapter 221'.

**.** According to an embodiment, the at least one sterile adapter 221, 221' comprises a projection 224 or skirt 224 facing said gap 235, 235'. Preferably, said annular skirt 224 extends around the rounded rim 223 and delimits at least a portion of said gap 235, 235'. Thereby, said at least one gap 235, 235' is delimited at least partially by the skirt 224 of the sterile adapter 221, 221'. According to an embodiment, said skirt 224 of the at least one sterile adapter 221, 221'cooperates with the at least one annular connector 212, 212', and preferably with said annular rim 213, 213' thereof, to form said labyrinth seal device. As mentioned above, each skirt 224 comprises a proximal surface 226, each proximal surface 226 distally delimiting a respective gap 235, 235'.

**.** According to an embodiment, said at least one sterile adapter 221, 221' comprises a distal coupling device 237 suitable to form a connection, and preferably a detachable connection, with a surgical instrument 240, 240' suitable to perform robotic surgery to a patient anatomy. According to an embodiment, said distal coupling device 237 of the at least one sterile adapter 221, 221' comprises means for snap-fit engaging a portion of said surgical instrument 240, 240', for example said means for snap-fit engaging comprises a pair of elastic elongated tongues 238 each forming a free end. According to an embodiment, said distal coupling device 237 of the at least one second component of sterile barrier 221 comprises a housing 239 for receiving a backend portion 241, 241' of said at least one surgical instrument 240, 240'. Thanks to the provision of said proximal coupling device 236 and of said distal coupling device 237, said at least one sterile adapter 221, 221' can detachably couple with a sterile surgical instrument 240, 240'.

**.** According to an embodiment, bearings 243 are provided within said labyrinth seal device 235.

**.** According to a general embodiment, a robotic surgery system 200 comprises at least one sterile barrier assembly 210 according to any one of the embodiments described above.

**.** Said robotic surgery system 200 further comprises at least one non-sterile robotic manipulator 220, 220' comprising a case 251, 251', and at least one roll motor 246, and at least one rolling body 230, 230' suitable to pivot in respect to said case 251, 251'.

**.** Said at least one connector 212, 212' of the surgical drape 201 of the sterile barrier assembly 210 is coupled with said case 251, 251' of the non-sterile robotic manipulator 220, 220'. The case 251, 251' preferably encases at least one motor, and preferably encases at least said roll motor 246.

**.** Said at least one sterile adapter 221, 221' of the sterile barrier assembly 210 is coupled with said at least one rolling body 230, 230' of the non-sterile robotic manipulator 220, 220'.

**.** Said at least one roll motor 246 is suitable to move at least said one rolling body 230, 230' together with said at least one sterile adapter 221. 221' of a pivoting movement with respect to said case 251, 251' of the non-sterile robotic manipulator 220, 220'.

**.** Said gap 235, 235' between the at least one connector 212, 212' of the surgical drape 201 and the at least one sterile adapter 221, 221' forms a rotatable seal, which prevents the non-sterile robotic manipulator to contaminate a sterile operatory field 255.

**.** According to a preferred embodiment, said at least one connector 212, 212' of the surgical drape 201 of the sterile barrier assembly 210 is coupled detachably and rigidly with said case 251, 251' of the non-sterile robotic manipulator 220, 220'.

**.** According to a preferred embodiment, said at least one sterile adapter 221, 221' of the sterile barrier assembly 210 is coupled detachably and rigidly with said at least one rolling body 230, 230' of the non-sterile robotic manipulator 220, 220'.

**.** According to a preferred embodiment, said rolling body 230, 230' houses at least one motor of the non-sterile robotic manipulator 220, 220' suitable for actuating a degree of freedom of a surgical instrument 240, 240' attached to the sterile adapter 221, 221'.

**.** According to an embodiment, said robotic surgery system 200 comprises a master console 244. According to an embodiment, said robotic surgery system 200 comprises a robotic slave assembly, suitable to be controlled by said master console 244, said robotic slave assembly comprising a robotic cart 225 and at least one robotic arm 211 extending from the cart 225 and defining a proximal-distal direction Z-Z. At least one electronic control device may be located inside the robotic cart 225.

**.** According to an embodiment, said robotic surgery system 200 comprises at least one surgical instrument 240, 240' comprising a instrument shaft 242 extending substantially along the proximal-distal direction Z-Z and having a proximal end and a distal end, a surgical end effector 245 at the distal end of the shaft 242 and a backend portion 241, 241' at or near the proximal end of the shaft 242. According to an embodiment, the backend portion 241, 241' is suitable to receive a pushing action transmitted through and across the second sheet 222 of the second component of sterile barrier 210. According to an embodiment, the backend portion 241, 241' comprises a plurality of transmission rods or the like, suitable to be pushed in order to actuate at least one actuation cable [not shown] of the surgical instrument to in turn actuate the end effector 245, for example a surgical wrist 245. According to an embodiment, said transmission rods are linearly displaceable.

**.** According to an embodiment, said sterile barrier assembly 210 is located proximally to, in other words upstream, said at least one surgical instrument 240, 240'. Thereby said sterile barrier assembly 210 is located upstream the backend 241, 241' of the at least one surgical instrument 240, 240'. According to an embodiment, said sterile barrier assembly 210 is located proximally to, in other words upstream, a shaft 242 of said at least one surgical instrument 240, 240'.

**.** According to an embodiment, said robotic cart 225 comprises at least a ground contact unit 252, for example a wheel 252, suitable to support said robotic cart 225 resting on a floor of an operating arena. According to an embodiment, said at least one ground contact unit 252 is not draped by said surgical drape 201.

**.** According to an embodiment, said at least one robotic arm 221 comprises a plurality of arm links 219, 219', 219", 219‴ jointed one another in respective joints forming a kinematic chain. Said robotic arm 211 is preferably a passive arm devoid of motors designed to be moved by a human action, for example pulled by an operator. Said robotic arm 211 may comprise brake systems at the joint of said arm links 219, 219', 219", 219'". According to an embodiment, one of said arm links 219, 219', 219", 219‴ is connected to at least two motorized robotic manipulators 220, 220', suitable to manipulate a robotic end effector, for example a wrist 245 of a surgical instrument 240, 240', within a patient's body along a plurality of degrees of freedom.

**.** Said robotic surgery system 200 comprises said at least one rolling body 230. 230'.

**.** According to an embodiment, said rolling body 230, 230' comprises, or is coupled to, said second component of sterile barrier 221, so that said second component of sterile barrier 221 moves integrally with said rolling body 230, 230'.

**.** According to an embodiment, each motorized manipulator 220, 220' comprises a case 251, 251' and at least a motor 246, 247.

**.** According to an embodiment, said at least one motor 246, 247 of the manipulator 220, 220' comprises at least one roll motor 246, suitable to move a rolling body 230, 230' of a rolling movement about a rolling axis Z-Z in respect of said case 251, 251'.

**.** According to a preferred embodiment, said at least one annular connector 212, 212' of the sterile barrier assembly 210 couples with said case 251, 251' of the manipulator 220, 220' and said at least one second component of sterile barrier 221 couples to said at least one rolling body 230, 230'. Bearings 243 may be provided between said at least one rolling body 230, 230' and said case 251, 251' of the manipulator 220, 220'. Preferably, said counter-coupling portion 217 is located on said case 251, 251', ad preferably on a distal portion of said case 251, 251'.

**.** According to an embodiment, said at least one motor 246, 247 of the manipulator 220, 220' further comprises at least one linear motor 247 suitable to move said rolling body 230, 230' along at least one linear direction, for example along the vertical direction, and preferably is suitable for moving said rolling body 230, 230' along three linear directions substantially orthogonal one another in respect of said case 251, 251'. Preferably, said at least one linear motor 247 acts proximally, in other words upstream, to said rolling motor 246. According to an embodiment, said at least one manipulator 220, 220' comprises three linear guiding devices 248, 249, 250 coupled to said at least one linear motor 247, and preferably each one coupled to a linear motor, and suitable for guiding the linear movement of the rolling body 230, 230' along three orthogonal directions, and at least one roll transmission element, for example a transmission belt, suitable to transmit a rolling action R from said roll motor 246 to said at least one rolling body 230, 230'.

**.** According to a preferred embodiment, motors 246, 247 are all provided proximally to, in other words upstream, the sterile barrier assembly 210.

**.** According to a preferred embodiment, said at least one surgical instrument 240, 240' is coupled to said rolling body 230. 230'.

**.** According to an embodiment, an anti-rotation system is provided designed to avoid relative rotation of the rolling body 230, 230' and the case 251, 251' of the manipulator 220, 220' during the coupling of the second component of sterile barrier 221 to said rolling body 230. 230'. According to an embodiment, said anti-rotation system comprises a radially cantilevered element, radially protruding away to the rotation axis of the rolling body 230, 230', to abut against a radially inward block tooth of the case 251, 251'.

**.** According to an embodiment, there are two motorized manipulators 220, 220' attached to a same link of a robotic arm 221, each manipulator 220, 220' has three Cartesian degrees of freedom, the three Cartesian degrees of freedom of one manipulator 220 are parallel to the three Cartesian degrees of freedom of the other manipulator 220'; downstream each manipulator 220, 220' is provided a roll motor 246 for moving a rolling body 230, 230' attached thereto of a rolling movement about a rolling axis Z-Z in respect of said case 251, 251'. Preferably, downstream the rolling body 230, 230' and preferably integral thereto, there is a instrument shaft having at its distal end an articulated end effector, for example having a pitch, a yaw and a grip degrees of freedom.

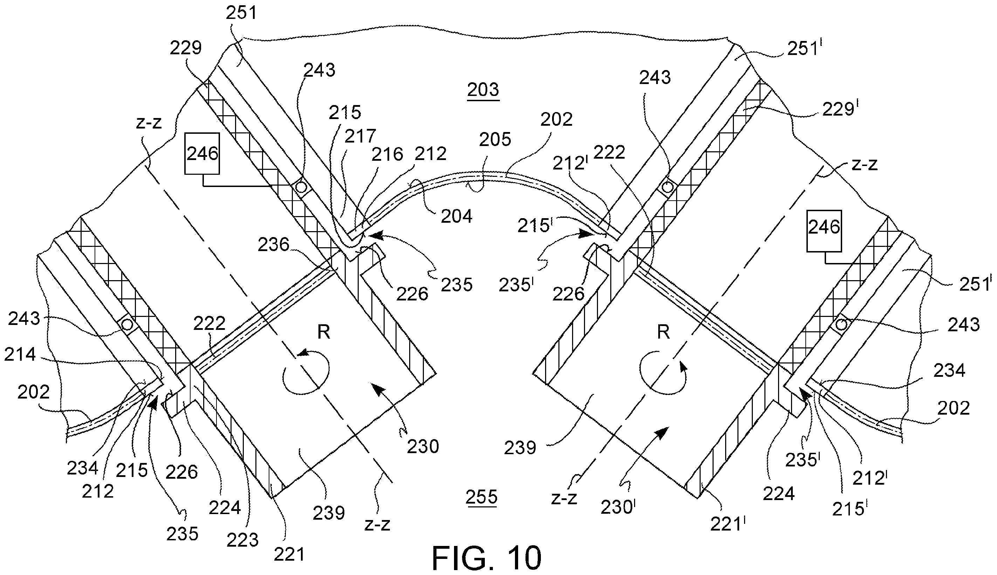

**.** According to a preferred embodiment, and as mentioned above, shown for example in **figure 10****,** the gap 235, 235' is between the proximal surface 226 of the skirt 224 of the sterile adapter 221, 221' and the distal surface 215, 215' of the connector 212, 212' of the surgical drape 201, wherein the sterile adapter 221, 221' is detachably connected to the manipulator connecting element 229, 229', and wherein the connector 212, 12' is detachably connected to the case 215. Thereby the sterile adapter 221, 221' and the manipulator connecting element 229, 229' together rotate with respect to the surgical drape 201, and at the same time the sterile adapter 221, 221' can be detached from the manipulator connecting element 229, 229' without requiring any action onto the surgical drape 201 nor on the connector 212, 212' of the surgical drape 201. Thereby, the sterile adapter 221, 221' can be detached from, and/or connected to, the manipulator connecting element 229, 229' also during surgery without for this reason compromising the integrity of the surgical drape 201, and in some embodiment event without touching any part of the surgical drape 201.

**.** According to a preferred embodiment, the sterile adapter 221, 221' is mounted to the robotic manipulator and is allowed to rotate with respect to the case 251, 251' without being received within the case 251, 251'. Rather the sterile adapter 221, 221', although comprising a membrane 222 which forms part of the sterile barrier assembly 210, is distal to the surgical drape 201.

**.** According to an embodiment non necessarily combinable with the embodiments described above, a sterile adapter 221, 221' for a robotic surgery system 200 is suitable to transmit a plurality of linear actuation actions and a roll action from a non-sterile robotic manipulator 220, 220' to a sterile surgical instrument 240, 240' having a backend 241 and a shaft 242 extending from said backend portion 241, wherein the sterile adapter 221, 221' comprises:
- a frame 223 for transmitting the roll action from the non-sterile robotic manipulator 220, 220' to the sterile surgical instrument 240, 240', wherein the frame 223 comprises a proximal coupling device 236, for coupling with the non-sterile robotic manipulator 220, 220', a distal coupling device 237, for coupling with the sterile surgical instrument 240, 240', wherein said frame 223 delimits a through opening between said proximal coupling device 236 and said distal coupling device 237; and
- a membrane 222 fixed to said frame 223 for transmitting through the thickness thereof the plurality of localized linear actuation actions from the non-sterile robotic manipulator 220, 220' to the sterile surgical instrument 240, 240', said membrane 222 is elastically stretchable with the purpose to result elastically biased towards a substantially flat configuration thereof; and
wherein said stretchable membrane 222 seals said through opening forming a distal cavity 239 between said stretchable membrane 222 and said distal coupling device 237, and wherein said distal cavity 239 is suitable for receiving at least a portion of the backend 241 of the sterile surgical instrument, and wherein said distal coupling device 237 comprises at least one abutment surface facing said stretchable membrane 222 and thereby at least partially delimiting said distal cavity 236, and wherein said at least one abutment surface is suitable for a portion of the surgical instrument 240, 240', such as the backend 241, to abut thereon.

**.** The non-sterile robotic manipulator 220, 220' may comprise motors for exerting said plurality of linear displacement actions, for example motors actuating a plurality of pistons, each piston exerting one of said linear displacement actions on the same membrane 222 of the sterile adapter 221, 221'.

**.** According to an embodiment, said distal cavity 239 includes lateral guiding surfaces apt to mate with at least one lateral countersurface of said sterile surgical instrument 240, 240', and wherein preferably said lateral guiding surfaces of the distal cavity 239 of the sterile adapter 221, 221' is substantially flat.

**.** According to an embodiment said distal cavity 239 includes a lateral opening designed for inserting said sterile surgical instrument 240, 240' into the sterile adapter 221, 221'.

**.** According to an embodiment, said distal coupling device 237 is designed to snap-fit engage with a portion of a surgical instrument 240, 240'.

**.** According to an embodiment, said distal cavity 239 includes a second lateral opening suitable to access the distal cavity 239 with the purpose of pushing said sterile surgical instrument 240, 240' out from said distal cavity 239 of the sterile adapter 221, 221'.

**.** According to an embodiment, said membrane 222 is in single flat piece.

**.** According to an embodiment, said distal coupling device 239 defines a distal seat having a distal through opening that opens distally outside of the distal cavity.

**.** According to an embodiment, said distal through opening is substantially aligned with the through opening that is sealed by the stretchable membrane 222, and preferably is coaxial with the through opening that is sealed by the stretchable membrane 222.

**.** According to an embodiment, said at least one abutment surface is beneath the stretchable membrane 222, and preferably under the encumber of the membrane 222.

**.** According to an embodiment, said frame 223 rigidly determines the reciprocal positioning and orientation of said proximal coupling device 236 and said distal coupling device 237, and preferably of the lateral opening of the distal coupling device 236.

**.** According to an embodiment, the sterile adapter 221, 221' comprises a spring-loaded engaging pin 253 and the manipulator connecting element 229 ,229' comprises a respective spring-loaded counter-engaging pin 259, to flag when the sterile adapter 221, 221' is coupled to the manipulator connecting element 229, 229', as previously described.

**.** In the following will be described a method for mounting a sterile barrier assembly, and will be described a method for dismounting a sterile barrier assembly.

**.** A method of mounting and/or dismounting a sterile barrier assembly of a robotic surgery system comprises the following steps:
- connecting and/or disconnecting at least one connector 212, 212' of a surgical drape 201 to at least one case 251, 251' of a non-sterile robotic manipulator 220, 220';
- connecting and/or disconnecting a sterile adapter 221, 221' to a rolling body 230, 230' of the non-sterile robotic manipulator 220, 220'.

**.** According to a mode of operation, the step of connecting and/or disconnecting a sterile adapter 221, 221' does not involve any action to the sterile drape 201.

**.** According to a mode of operation, the step of connecting comprises moving proximally the sterile adapter 221, 221', for example of a rotative motion such as to screw the adapter to the rolling body 230, 230', either directly or indirectly by means of interposition of a manipulator connecting element 229, 229'. When it is provided a manipulator connecting element 229, 229' interposed between the rolling body 230, 230' and the sterile adapter 221, 221', the step of connecting comprises moving proximally the sterile adapter 221, 221', for example of a rotative motion such as to screw the adapter 221, 221' to the manipulator connecting element 229, 229'.

**.** According to a mode of operation, the step of disconnecting comprises moving proximally the sterile adapter 221, 221', for example of a rotative motion such as to unscrew the adapter to the rolling body 230, 230'. When it is provided a manipulator connecting element 229, 229' interposed between the rolling body 230, 230' and the sterile adapter 221, 221', the step of disconnecting comprises moving distally the sterile adapter 221, 221', for example of a rotative motion such as to unscrew the adapter 221, 221' from the manipulator connecting element 229, 229'.

**.** According to a mode of operation, the method comprises firstly connecting the connector 212, 212' of the surgical drape 201 to the case 251, 251' of the manipulator 220, 220', and subsequently connecting the sterile adapter 221, 221' to the rolling body 230, 230' of the robotic manipulator 220, 220'. When it is provided a manipulator connecting element 229, 229' interposed between the rolling body 230, 230' and the sterile adapter 221, 221', the step of connecting comprises firstly connecting the connector 212, 212' of the surgical drape 201 to the case 251, 251' of the manipulator 220, 220', and subsequently connecting the sterile adapter 221, 221' to the manipulator connecting element 229, 229' attached to the rolling body 230, 230' of the robotic manipulator 220, 220'. Attaching the manipulator connecting element 229, 229' to the rolling body 230, 230' may be carried out before connecting the connector 212, 212' of the surgical drape 201 to the case 251, 251', so that connecting the connector 212, 212' of the surgical drape 201 to the case 251, 251' may be carried out after having attached the manipulator connecting element 229, 229' to the rolling body 230, 230'.

**.** According to a mode of operation, the step of disconnecting comprises firstly disconnecting the sterile adapter 221, 221' from the rolling body 230, 230' of the robotic manipulator 220, 220', and subsequently disconnecting the connector 212, 212' of the surgical drape 201 from the case 251, 251'. When it is provided a manipulator connecting element 229, 229' interposed between the rolling body 230, 230' and the sterile adapter 221, 221', the step of disconnecting comprises firstly disconnecting the sterile adapter 221, 221' from the manipulator connecting element 229, 229' attached to the rolling body 230, 230' of the robotic manipulator 220, 220', and subsequently disconnecting the connector 212, 212' of the surgical drape 201 from the case 251, 251'. The method may also comprise the step of disattaching (i.e. disconnecting) the manipulator connecting element 229, 229' from the rolling body 230, 230', this step may be carried out after having disconnected the connector 212, 212' of the surgical drape 201 from the case 251, 251'.

**.** Thereby, the sterile adapter 212, 212' may be the last component of the sterile barrier assembly to be connected.

**.** Thereby, the sterile adapter 212, 212' may be the first component of the sterile barrier assembly to be disconnected.

**.** According to a mode of operation, the step of connecting and/or disconnecting a sterile adapter 221, 221' is carried out during surgery.

**.** In the light of the above, a method for mounting a sterile barrier assembly may comprise the steps of: firstly connecting at least one connector 212, 212' of a surgical drape 201 to at least one case 251, 251' of the robotic manipulator 220, 220', and subsequently connecting at least one sterile adapter 221, 221' to the rolling body 230, 230' of the at least one robotic manipulator 220, 220', wherein the at least one robotic manipulator 220, 220' having its case 250, 251' already connected to the at least one connector 212, 212' of a surgical drape 201. When two robotic manipulators 220, 220' are provided, a skilled in the art will understand that the method for mounting may comprise firstly connecting the connector 212, 212' to the case 251, 251' of either manipulator 220, 220', and subsequently connecting the respective sterile adapter 221, 221'.

**.** In the light of the above, a method for dismounting a sterile barrier assembly may comprise the steps of: firstly disconnecting at least one sterile adapter 221, 221' from the rolling body 230, 230' of the at least one robotic manipulator 220, 220', wherein the at least one robotic manipulator 220, 220' having its case 250, 251' connected to the at least one connector 212, 212' of a surgical drape 201, and subsequently disconnecting the at least one connector 212, 212' of the surgical drape 201 from at least one case 251, 251' of the robotic manipulator 220, 220'. When two robotic manipulators 220, 220' are provided, a skilled in the art will understand that the method for dismounting may comprise firstly disconnecting the sterile adapter 221, 221' from either manipulator 220, 220', and subsequently disconnecting the connector 212, 212' from the case 251, 251' of the respective manipulator 220, 220'.

**.** According to a mode of operation, the method is carried out by means of a sterile barrier assembly and/or a robotic surgery system according to any of the embodiments described above.

**.** By virtue of the features described above, provided either independently or in combination in particular embodiments, it is possible to meet the above mentioned needs providing the above cited advantages, and in particular:
- it is provided a sterile barrier assembly 210 capable to prevent contamination of the sterile operatory field 255 and at the same time able to decouple the rotatable seal so that to avoid local frictional wear onto the sterile adapter 221, 221' and/or to the connector 212, 212' of the surgical drape 201;
- the sterile adapter 221, 221' and the connector 212, 212' may be spaced in the proximal - distal direction, so that the sterile adapter frame 223 is distal to the respective connector 212, 212' of the surgical drape 201;
- the sterile adapter 221, 221' may be detached from the robot, without the need of detaching the surgical drape 201;
- at the same time the connector of the surgical drape 201 may be disconnected from the case 251, 251' of the robot without for this reason requiring to detach the sterile adapter 221, 221'.
- motors can be located upstream the rotatable seal of the sterile barrier assembly;
- it is provided a surgical drape able to receive a rolling body without for this reason incur in structural damages or plastic deformations;
- it is provided a sterile barrier assembly made of two separate pieces with a gap interposed there between, allowing relative rolling movement between said two separate pieces;
- it is provided a sterile barrier assembly with coupling means to form a reversible connection with at least one surgical instrument;
- a single surgical drape may be employed to drape a robotic arm and a robotic cart, thereby reducing the number of components of the robotic surgery system and thereby reducing the time needed for draping.

**.** Those skilled in art may make many changes and adaptations to the embodiments described above or may replace elements with others which are functionally equivalent in order to satisfy contingent needs without however departing from the scope of the appended claims.

### LIST OF REFERENCES

| | |
|---|---|
| **200** | Robotic surgery system |
| **201** | Surgical drape or sterile drape |
| **202** | Flexible body of the surgical drape |
| **203** | Through cavity |
| **204** | First inner surface |
| **205** | Second outer surface |
| **206** | Proximal opening edge |
| **207** | Proximal opening |
| **208** | Distal opening edge, or first distal opening edge |
| **208'** | Distal opening edge, or second distal opening edge |
| **209** | Drape distal opening, or first distal opening |
| **209'** | Drape distal opening, or second distal opening |
| **210** | Sterile barrier assembly |
| **211** | Robotic arm |
| **212** | Connector, or annular connector, or first annular connector |
| **212'** | Connector, or annular connector, or second annular connector |
| **213** | Annular rim of the first annular connector |
| **213'** | Annular rim of the second annular connector |
| **214** | Inner surface of the annular rim of the first annular connector |
| **214'** | Inner surface of the annular rim of the second annular connector |
| **215** | Distal surface of the annular rim of the first annular connector |
| **215'** | Distal surface of the annular rim of the second annular connector |
| **216** | Coupling device of the annular connector of the surgical drape |
| **217** | Counter-coupling portion of the robotic surgical system |
| **218** | Proximal coupling interface of the surgical drape |
| **219**, **219', 219", 219"',** | Arm links of the robotic arm |
| **220, 220'** | Manipulator of the robotic surgery system, or robotic motorized manipulator |
| **221, 221'** | Sterile adapter |
| **222** | Second sheet of the sterile barrier assembly |
| **223** | Frame or rounded rim of the adapter |
| **224** | Skirt of the adapter |
| **225** | Cart of the robotic surgery system |
| **226** | Proximal surface of the adapter |
| **228** | Proximal opening of second sheet |
| **229** | Manipulator connecting element |
| **230, 230'** | Rolling body |
| **231, 231'** | Pouch of the surgical drape |
| **232** | Bridge portion of the body of the surgical drape |
| **233** | Base portion of the body of the surgical drape |
| **234** | Proximal annular surface of the annular connector |
| **235, 235'** | Gap |
| **236** | Proximal coupling device of the adapter |
| **237** | Distal coupling device of the adapter |
| **238** | Elongated tongue, or elastic tongue |
| **239** | Housing of the adapter |
| **240, 240'** | Surgical instrument |
| **241** | Backend of surgical instrument |
| **242** | Shaft of surgical instrument |
| **243** | Bearings |
| **244** | Master controller |
| **245** | Wrist of the surgical instrument, or end effector |
| **246** | Roll motor |
| **247** | Linear motor |
| **248, 248', 248"** | Linear guiding device |
| **251, 251'** | Case, or protective case of the robotic manipulator |
| **252** | Ground contact unit |
| **254, 254'** | Proximal pouch opening |
| **255** | Sterile operatory field |
| **256, 256'** | Center of the connector |
| **257, 257'** | Center of the adapter frame |
| **z-z** | Axis |
| **R** | Roll direction |

## Claims

1. Sterile barrier assembly (210) for a robotic surgery system (200) comprising:
- a surgical drape (201) having at least one drape opening (209, 209');
- at least one connector (212, 212') contouring said at least one drape opening (209, 209') and suitable for coupling with a case (251, 251') of a non-sterile robotic manipulator (220, 220'); and
- at least one sterile adapter (221, 221') having a frame (223) comprising a coupling device (237) for coupling with at least one surgical instrument (240, 240'), said at least one sterile adapter (221, 221') being suitable to be coupled with at least one rolling body (230, 230') of the non-sterile robotic manipulator (220, 220'), wherein said at least one rolling body (230, 230') is suitable to pivot in respect to said case (251, 251');
wherein
- the at least one connector (212, 212') of the surgical drape (201) and the at least one sterile adapter (221, 221') are made in separate pieces;
- the at least one sterile adapter (221, 221') is suitable to pivot in respect of the at least one connector (212, 212') of the surgical drape (201);
- between the at least one connector (212, 212') of the surgical drape (201) and the at least one sterile adapter (221, 221') it is provided a gap (235, 235'); and wherein
said at least one connector (212, 212') of the surgical drape (201) comprises a distal surface (215, 215') facing distally and said sterile adapter (221, 221') comprises a proximal surface (226) facing proximally,
said distal surface (215, 215') of the connector (212, 212') faces said proximal surface (226) of the sterile adapter (221, 221') delimiting said gap (235, 235');
said gap (235, 235') prevents the non-sterile robotic manipulator to contaminate a sterile operatory field (255) while avoids direct connection of the sterile adapter (221, 221') to the connector (212, 212') of the surgical drape (201),
said gap (235, 235') forms together with said at least one connector (212, 212') and with said at least one sterile adapter (221, 221') a rotatable seal, which avoids generating rotative friction onto the at least one connector and the at least one sterile adapter, while preserving sterility of the sterile operatory field.

2. Sterile barrier assembly (210) according to claim 1, wherein said at least one connector (212, 212') of the surgical drape (201) has an annular rim (213, 213') and said at least sterile adapter (221, 221') has an annular frame so that said gap (235, 235') is an annular gap.

3. Sterile barrier assembly (210) according to claim **2,** wherein the annular rim (213, 213') and the annular frame (223) of the sterile adapter (221, 221') are arranged coaxially.

4. Sterile barrier assembly (210) according to any one of the preceding claims, wherein the sterile adapter (221, 221') comprises a projection (224) or skirt (224) facing proximally said gap (235, 235'), said skirt (224) comprising said proximal surface (226).

5. Sterile barrier assembly (210) according to any one of the preceding claims, wherein said sterile drape (201) comprises:
- a pair of drape openings (209, 209');
- a pair of connectors (212, 212') each contouring a respective drape opening (209, 209');
- a pair of sterile adapters (221, 221') each having a frame (223) comprising a coupling device (237) for coupling with a respective surgical instrument (240, 240');
wherein:
- each connector (212, 212') of the surgical drape (201) and the respective sterile adapter (221, 221') are made in separate pieces;
- each sterile adapter (221, 221') is suitable to pivot in respect of the respective connector (212, 212') of the surgical drape (201);
- between each connector (212, 212') of the surgical drape (201) and the respective sterile adapter (221, 221') it is provided a gap (235, 235').

6. Sterile barrier assembly (210) according to claim **5,** wherein the surgical drape (201) is in single piece.

7. Sterile barrier assembly (210) according to claim **5** or **6,** wherein the surgical drape (201) comprises a pair of pouches (231, 231'), each pouch (231, 231') delimiting a respective drape opening (209, 209'), each pouch (231, 231') being suitable for draping a one non-sterile robotic manipulator (220, 220').

8. Robotic surgery system (200) comprising:
- at least one sterile barrier assembly (210) according to any one of the preceding claims;
- at least one non-sterile robotic manipulator (220, 220') comprising:
- a case (251, 251'), and
- at least one roll motor (246), and
- at least one rolling body (230, 230') suitable to pivot in respect to said case (251, 251');
wherein:
- said at least one connector (212, 212') of the surgical drape (201) of the sterile barrier assembly (210) is coupled with said case (251, 251') of the non-sterile robotic manipulator (220, 220');
- said at least one sterile adapter (221, 221') of the sterile barrier assembly (210) is coupled with said at least one rolling body (230, 230') of the non-sterile robotic manipulator (220, 220');
- said at least one roll motor (246) is suitable to move at least said one rolling body (230, 230') together with said at least one sterile adapter (221, 221') of a pivoting movement with respect to said case (251, 251') of the non-sterile robotic manipulator (220, 220')

9. Robotic surgery system (200) according to claim **8,** wherein said at least one connector (212, 212') of the surgical drape (201) of the sterile barrier assembly (210) is coupled detachably and rigidly with said case (251, 251') of the non-sterile robotic manipulator (220, 220').

10. Robotic surgery system (200) according to claim **8** or **9,** wherein said at least one sterile adapter (221, 221') of the sterile barrier assembly (210) is coupled detachably and rigidly with said at least one rolling body (230, 230') of the non-sterile robotic manipulator (220, 220').

11. Robotic surgery system (200) according to claim **8. 9** or **10,** wherein said rolling body of the non-sterile robotic manipulator (220, 220') comprises a manipulator connecting element (229, 229'), and wherein said sterile adapter (221, 221') is detachably engaged with said manipulator connecting element (229, 229'), and wherein the engagement between said sterile adapter (221, 221') and said manipulator connecting element (229, 229') is distal to said case (215) and/or distal to said connector (212, 212') of the surgical drape (201).

## Patentansprüche

1. Sterile Barriereanordnung (210) für ein robotergestütztes Chirurgiesystem (200), umfassend:
- ein chirurgisches Abdecktuch (201), welches mindestens eine Abdecktuchöffnung (209, 209') aufweist;
- mindestens einen Verbinder (212, 212'), welcher die mindestens eine Abdecktuchöffnung (209, 209') konturiert und geeignet zur Kopplung mit einem Gehäuse (251, 251') eines nicht-sterilen Roboter-Manipulators ist (220, 220');
und
- mindestens einen sterilen Adapter (221, 221'), welcher einen Rahmen (223) aufweist, umfassend eine Kopplungsvorrichtung (237) zum Koppeln mit mindestens einem chirurgischen Instrument (240, 240'), wobei der mindestens eine sterile Adapter (221, 221') geeignet ist, um mit mindestens einem Rollkörper (230, 230') des nicht-sterilen Roboter-Manipulators (220, 220') gekoppelt zu werden, wobei der mindestens eine Rollkörper (230, 230') geeignet ist, um in Bezug auf das Gehäuse (251, 251') zu schwenken;
wobei
- der mindestens eine Verbinder (212, 212') des chirurgischen Abdecktuchs (201) und der mindestens eine sterile Adapter (221, 221') aus getrennten Stücken hergestellt sind;
- der mindestens eine sterile Adapter (221, 221') geeignet ist, um in Bezug auf den mindestens einen Verbinder (212, 212') des chirurgischen Abdecktuchs (201) zu schwenken;
- zwischen dem mindestens einen Verbinder (212, 212') des chirurgischen Abdecktuchs (201) und dem mindestens einen sterilen Adapter (221, 221') ein Spalt (235, 235') bereitgestellt ist; und wobei
der mindestens eine Verbinder (212, 212') des chirurgischen Abdecktuchs (201) eine distale Oberfläche (215, 215') umfasst, welche distal ausgerichtet ist, und der sterile Adapter (221, 221') eine proximale Oberfläche (226) umfasst, welche proximal ausgerichtet ist,
wobei die distale Oberfläche (215, 215') des Verbinders (212, 212') gegenüber der proximalen Oberfläche (226) des sterilen Adapters (221, 221') ausgerichtet ist, wobei sie den Spalt (235, 235') begrenzt;
der Spalt (235, 235') verhindert, dass der nicht-sterile Roboter-Manipulator ein steriles Operationsfeld (255) kontaminiert, während eine direkte Verbindung des sterilen Adapters (221, 221') zu dem Verbinder (212, 212') des chirurgischen Abdecktuchs (201) vermieden wird, wobei
der Spalt (235, 235') zusammen mit dem mindestens einen Verbinder (212, 212') und mit dem mindestens einen sterilen Adapter (221, 221') eine rotierbare Dichtung bildet, welche es vermeidet, Rotationsreibung an dem mindestens einen Verbinder und dem mindestens einen sterilen Adapter zu erzeugen, während die Sterilität des sterilen Operationsfeldes bewahrt wird.

2. Sterile Barriereanordnung (210) nach Anspruch 1, wobei der mindestens eine Verbinder (212, 212') des chirurgischen Abdecktuchs (201) einen ringförmigen Rand (213, 213') aufweist und der mindestens eine sterile Adapter (221, 221') einen ringförmigen Rahmen aufweist, so dass der Spalt (235, 235') ein ringförmiger Spalt ist.

3. Sterile Barriereanordnung (210) nach Anspruch **2,** wobei der ringförmige Rand (213, 213') und der ringförmige Rahmen (223) des sterilen Adapters (221, 221') koaxial angeordnet sind.

4. Sterile Barriereanordnung (210) nach einem der vorhergehenden Ansprüche, wobei der sterile Adapter (221, 221') einen Vorsprung (224) oder eine Schürze (224) umfasst, welcher/welche proximal gegenüber dem Spalt (235, 235') ausgerichtet ist, wobei die Schürze (224) die proximale Oberfläche (226) umfasst.

5. Sterile Barriereanordnung (210) nach einem der vorhergehenden Ansprüche, wobei das sterile Abdecktuch (201) umfasst:
- ein Paar von Abdecktuchöffnungen (209, 209');
- ein Paar von Verbindern (212, 212'), welche jeweils eine entsprechende Abdecktuchöffnung (209, 209') konturieren;
- ein Paar von sterilen Adaptern (221, 221'), welche jeweils einen Rahmen (223) aufweisen, umfassend eine Kopplungsvorrichtung (237) zum Koppeln mit einem entsprechenden chirurgischen Instrument (240, 240');
wobei:
- jeder Verbinder (212, 212') des chirurgischen Abdecktuchs (201) und der jeweilige sterile Adapter (221, 221') aus getrennten Stücken hergestellt sind;
- jeder sterile Adapter (221, 221') geeignet ist, in Bezug auf den jeweiligen Verbinder (212, 212') des chirurgischen Abdecktuchs (201) zu schwenken;
- zwischen jedem Verbinder (212, 212') des chirurgischen Abdecktuchs (201) und dem jeweiligen sterilen Adapter (221, 221') ein Spalt (235, 235') bereitgestellt ist.

6. Sterile Barriereanordnung (210) nach Anspruch **5,** wobei das chirurgische Abdecktuch (201) in einem einzigen Stück ist.

7. Sterile Barriereanordnung (210) nach Anspruch **5** oder **6,** wobei das chirurgische Abdecktuch (201) ein Paar von Beuteln (231, 231') umfasst, wobei jeder Beutel (231, 231') eine jeweilige Abdecktuchöffnung (209, 209') begrenzt, wobei jeder Beutel (231, 231') zum Abdecken eines nicht-sterilen Roboter-Manipulators (220, 220') geeignet ist.

8. Robotergestütztes Chirurgiesystem (200) umfassend:
- mindestens eine sterile Barriereanordnung (210) nach einem der vorhergehenden Ansprüche;
- mindestens einen nicht-sterilen Roboter-Manipulator (220, 220') umfassend:
- ein Gehäuse (251, 251'), und
- mindestens einen Rollmotor (246), und
- mindestens einen Rollkörper (230, 230'), welcher geeignet ist, in Bezug auf das Gehäuse (251, 251') zu schwenken;
wobei:
- der mindestens eine Verbinder (212, 212') des chirurgischen Abdecktuchs (201) der sterilen Barriereanordnung (210) mit dem Gehäuse (251, 251') des nicht-sterilen Roboter-Manipulators (220, 220') gekoppelt ist;
- der mindestens eine sterile Adapter (221, 221') der sterilen Barriereanordnung (210) mit dem mindestens einen Rollkörper (230, 230') des nicht-sterilen Roboter-Manipulators (220, 220') gekoppelt ist;
- der mindestens eine Rollmotor (246) geeignet ist, um mindestens den einen Rollkörper (230, 230') zusammen mit dem mindestens einen sterilen Adapter (221, 221') in einer Schwenkbewegung in Bezug auf das Gehäuse (251, 251') des nicht-sterilen Roboter-Manipulators (220, 220') zu bewegen.

9. Robotergestütztes Chirurgiesystem (200) nach Anspruch **8,** wobei der mindestens eine Verbinder (212, 212') des chirurgischen Abdecktuchs (201) der sterilen Barriereanordnung (210) lösbar und starr mit dem Gehäuse (251, 251') des nicht-sterilen Roboter-Manipulators (220, 220') gekoppelt ist.

10. Robotergestütztes Chirurgiesystem (200) nach Anspruch **8** oder **9,** wobei der mindestens eine sterile Adapter (221, 221') der sterilen Barriereanordnung (210) lösbar und starr mit dem mindestens einen Rollkörper (230, 230') des nicht-sterilen Roboter-Manipulators (220, 220') gekoppelt ist.

11. Robotergestütztes Chirurgiesystem (200) nach Anspruch **8, 9** oder **10,** wobei der Rollkörper des nicht-sterilen Roboter-Manipulators (220, 220') ein Manipulator-Verbindungselement (229, 229') umfasst, und wobei der sterile Adapter (221, 221') lösbar mit dem Manipulator-Verbindungselement (229, 229') in Eingriff steht, und wobei der Eingriff zwischen dem sterilen Adapter (221, 221') und dem Manipulator-Verbindungselement (229, 229') distal zu dem Gehäuse (215) und/oder distal zu dem Verbinder (212, 212') des chirurgischen Abdecktuchs (201) erfolgt.

## Revendications

1. Ensemble barrière stérile (210) pour un système de chirurgie robotisé (200) comprenant :
- une enveloppe chirurgicale (201) ayant au moins une ouverture d'enveloppe (209, 209') ;
- au moins un connecteur (212, 212') entourant ladite au moins une ouverture d'enveloppe (209, 209') et convenant pour une connexion avec un boîtier (251, 251') d'un manipulateur robotisé (220, 220') non stérile ; et
- au moins un adaptateur stérile (221, 221') ayant un cadre (223) comprenant un dispositif d'accouplement (237) pour un accouplement avec au moins un instrument chirurgical (240, 240'), ledit au moins un adaptateur stérile (221, 221') convenant pour être accouplé avec au moins un corps roulant (230, 230') du manipulateur robotisé (220, 220') non stérile, dans lequel ledit au moins un corps roulant (230, 230') convient pour pivoter par rapport audit boîtier (251, 251') ;
dans lequel
- ledit au moins un connecteur (212, 212') de l'enveloppe chirurgicale et ledit au moins un adaptateur stérile (221, 221 ') sont réalisés comme pièces individuelles ;
- ledit au moins un adaptateur stérile (221, 221') est adapté pour pivoter par rapport audit au moins un connecteur (212, 212') de l'enveloppe chirurgicale (201) ;
- entre ledit au moins un connecteur (212, 212') de l'enveloppe chirurgicale (201) et ledit au moins un adaptateur stérile (221, 221') un espace (235, 235') est prévu ; et dans lequel
ledit au moins un connecteur (212, 212') de l'enveloppe chirurgicale (201) comprend une surface distale (215, 215') avec une orientation distale et l'adaptateur stérile (221, 221') comprend une surface proximale (226) avec une orientation proximale,
ladite surface distale (215, 215') du connecteur (212, 212') est en regard de la surface proximale (226) de l'adaptateur stérile (221, 221'), délimitant ledit espace (235, 235') ;
ledit espace (235, 235') évite que le manipulateur robotisé non-stérile contamine un champ d'opération stérile (255) en évitant une connexion directe de l'adaptateur stérile (221, 221') au connecteur (212, 212') de l'enveloppe chirurgicale (201),
ledit espace (235, 235') forme, ensemble avec ledit au moins un connecteur (212, 212') et avec ledit au moins un adaptateur stérile (221, 221'), un joint tournant qui évite d'engendrer une friction rotative sur ledit au moins un connecteur et ledit au moins un adaptateur stérile, tout en conservant la stérilité du champ d'opération stérile.

2. Ensemble barrière stérile (210) selon la revendication 1, dans lequel ledit au moins un connecteur (212, 212') de l'enveloppe chirurgicale (201) a un bord annulaire (213, 213') et ledit au moins un adaptateur stérile (221, 221') a un cadre annulaire, si bien que ledit espace (235, 235') est un espace annulaire.

3. Ensemble barrière stérile (210) selon la revendication 2, dans lequel le bord annulaire (213, 213') et le cadre annulaire (223) de l'adaptateur stérile (221, 221') sont agencés coaxialement.

4. Ensemble barrière stérile (210) selon l'une des revendications précédentes, dans lequel l'adaptateur stérile (221, 221') comprend une projection (224) ou jupe (224) proximalement en regard dudit espace (235, 235'), ladite jupe (224) comprenant ladite surface proximale (226).

5. Ensemble barrière stérile (210) selon l'une des revendications précédentes, dans lequel ladite enveloppe stérile (201) comprend :
- une paire d'ouvertures d'enveloppe (209, 209') ;
- une paire de connecteurs (212, 212'), chacun contournant une ouverture d'enveloppe (209, 209') respective ;
- une paire d'adaptateurs stériles (221, 221'), chacun ayant un cadre (223) comprenant un dispositif d'accouplement (237) pour un accouplement a un instrument chirurgical respectif (240, 240') ;
dans lequel :
- chaque connecteur (212, 212') de l'enveloppe chirurgicale (201) et l'adaptateur stérile respectif (221, 221 ') étant réalisé comme pièces individuelles ;
- chaque adaptateur stérile (221, 221') est en mesure de pivoter par rapport au connecteur respectif (212, 212')
de l'enveloppe chirurgicale (201) ;
- entre chaque connecteur (212, 212') de l'enveloppe chirurgicale (201) et l'adaptateur stérile (221, 221') respectif, un espace (235, 235') est prévu.

6. Ensemble barrière stérile (210) selon la revendication 5, dans lequel l'enveloppe chirurgicale (201) est en une seule pièce.

7. Ensemble barrière stérile (210) selon la revendication 5 ou 6, dans lequel l'enveloppe chirurgicale (201) comprend une paire de pochettes (231, 231'), chaque pochette (231, 231') délimitant une ouverture d'enveloppe (209, 209') respective, chaque pochette (231, 231') convenant pour envelopper un manipulateur robotisé (220, 220') non stérile.

8. Système de chirurgie robotisé (200) comprenant :
- au moins un ensemble barrière stérile (210) selon l'une des revendications précédentes ;
- au moins un manipulateur robotisé (220, 220') non stérile comportant :
- un boîtier (251, 251') et
- au moins un moteur de roulage (246) et
- au moins un corps de roulage (230, 230') pouvant pivoter par rapport audit boîtier (251, 251') ;
dans lequel :
- ledit au moins un connecteur (212, 212') de l'enveloppe chirurgicale (201) de l'ensemble barrière stérile (210) est accouplé audit boîtier (251, 251') du manipulateur robotisé (220, 220') non stérile ;
- ledit au moins un adaptateur stérile (221, 221') de l'ensemble barrière stérile (210) est accouplé audit au moins un corps de roulage (230, 230') du manipulateur robotisé (220, 220') non stérile ;
- ledit au moins un moteur de roulage (246) est adapté pour déplacer au moins un corps de roulage (230, 230') ensemble avec ledit au moins un adaptateur stérile (221, 221') d'un mouvement pivotant par rapport audit boîtier (251, 251') du manipulateur robotisé (220, 220') non stérile.

9. Système de chirurgie robotisé (200) selon la revendication 8, dans lequel ledit au moins un connecteur (212, 212') de l'enveloppe chirurgicale (201) de l'ensemble barrière stérile (210) est accouplé de façon amovible et rigide audit boîtier (251, 251') du manipulateur robotisé (220, 220') non stérile.

10. Système de chirurgie robotisé (200) selon la revendication 8 ou 9, dans lequel ledit au moins un adaptateur stérile (221, 221') de l'ensemble barrière stérile (210) est accouplé de façon amovible et rigide audit au moins un corps de roulage (230, 230') du manipulateur robotisé (220, 220') non stérile.

11. Système de chirurgie robotisé (200) selon la revendication 8, 9 ou 10, dans lequel le corps de roulage du manipulateur robotisé (220, 220') non stérile comprend un élément de connexion de manipulateur (229, 229') et dans lequel l'adaptateur stérile (221, 221') est relié de façon amovible audit élément de connexion de manipulateur (229, 229'), et dans lequel la liaison entre l'adaptateur stérile (221, 221') et ledit élément de connexion de manipulateur (229, 229') est distal par rapport audit boîtier (215) et/ou distal par rapport audit connecteur (212, 212') de l'enveloppe chirurgicale (201).
